# EUROPEAN PATENT APPLICATION

(11) **EP 1 618 849 A1**
(43) Date of publication of application: **25.01.2006**
(21) Application number: 05004839.6
(22) Date of filing: 04.03.2005
(51) Int. Cl.: A61B 17/17, A61F 2/46

(54) **Navigated surgical sizing guide**

(30) Priority: 23.07.2004 US 897935
(71) Applicant: Zimmer Technology, Inc., Chicago, Illinois 60606 (US)
(72) Inventor: Grimm, James E., Winona Lake, IN 46590 (US)
(74) Representative: Manitz, Finsterwald & Partner GbR

(57) **Abstract**

The present invention provides a navigated surgical sizing guide for intraoperatively making surgical measurements.

## Description

### FIELD OF THE INVENTION

The present invention relates to surgical components used in conjunction with a surgical navigation system. In particular, the present invention relates to a navigated surgical sizing guide for intraoperatively making a surgical measurement.

Many surgical procedures are now performed with surgical navigation systems in which sensors detect tracking elements attached in known relationship to an object in the surgical suite such as a surgical instrument, implant, or patient body part. The sensor information is fed to a computer that then triangulates the three dimensional position of the tracking elements within the surgical navigation system coordinate system. Thus, the computer can resolve the position and orientation of the object and display the position and orientation for surgeon guidance. For example, the position and orientation can be shown superimposed on an image of the patient's anatomy obtained via X-ray, CT scan, ultrasound, or other imaging technology. Likewise, positional data may be provided in the form of textual or numerical readouts for surgeon reference.

### SUMMARY

The present invention provides a navigated surgical sizing guide for intraoperatively making a surgical measurement.

In one aspect of the invention, a navigated surgical sizing guide is provided for use with a surgical navigation system during an orthopaedic surgical procedure to make a measurement. The navigated surgical sizing guide includes first means for engaging a first position at a surgical site and second means for engaging a second position at a surgical site, the first and second means being mounted for relative translation. The guide further includes first means for being tracked by the surgical navigation system. The first means for being tracked is fixedly mounted to the first means for engaging such that the surgical navigation system is able to track the position of the first means for engaging and resolve and output the relative spacing between the first means for engaging and the second means for engaging.

In another aspect of the invention, a navigated surgical sizing guide is provided for use with a surgical navigation system during an orthopaedic surgical procedure to measure the size of a bone between first and second locations on the bone. The navigated surgical sizing guide includes a first probe for contacting the first location on the bone and a second probe for contacting the second location on the bone. The first probe includes a first tracking element trackable by the surgical navigation system and the second probe includes a second tracking element trackable by the surgical navigation system. The first and second probes are mounted for translation relative to one another such that they may be moved between a first position in which they are relatively near one another and a second position in which they are relatively far apart. The surgical navigation system is able to track the tracking elements and resolve the distance between the first and second probes.

In another aspect of the invention, a navigated surgical sizing guide is provide for use with a surgical navigation system during an orthopaedic surgical procedure to measure the anterior-posterior distance between the anterior femoral cortex and the posterior femoral condyles of a femur. The navigated surgical sizing guide includes a probe for contacting the anterior femoral cortex and a paddle for contacting the posterior femoral condyles. The probe and paddle each include a tracking element trackable by the surgical navigation system. The probe and paddle are mounted for linear translation relative to one another such that the probe and paddle may be moved between a first position in which they are relatively near one another and a second position in which they are relatively far apart.
The surgical navigation system is able to track the tracking elements and resolve the distance between the probe and paddle.

The invention can be used in a method of performing an orthopaedic surgical procedure at a surgical site of a patient's body, which includes: providing a navigated surgical sizing guide having first and second probes mounted for relative translation; positioning the navigated surgical sizing guide adjacent to a bone with the probes abutting spaced apart portions of the bone; and activating the surgical navigation system to determine the relative spacing of the probes.
Preferably, each of the first and second probes has a tracking element attached to it and the surgical navigation system tracks each tracking element to resolve the spacing between the first and second probes.
Preferably, positioning the navigated surgical sizing guide comprises abutting one probe against the anterior femoral cortex adjacent the distal end of a femur and abutting another probe against the posterior femoral condyles such that the surgical navigation system is able to determine the anterior-posterior size of the femur.
Preferably, the method further comprises establishing a datum relative to the surgical site with the orthopaedic guide; and engaging the datum with a surgical component to position the surgical component at a desired position relative to the surgical site.
Preferably, providing a navigated surgical sizing guide further comprises providing a body on which the first and second probes are mounted, the body and each probe being separately trackable by the surgical navigation system, the method further comprising: positioning the body at a known anterior-posterior distance from one of the first and second probes by tracking the body and probe with the surgical navigation system.
Preferably, establishing a datum comprises inserting a pin into the distal femoral bone and engaging the datum comprises engaging the datum with a femoral finishing guide to establish the anterior-posterior location of the femoral finishing guide.

### BRIEF DESCRIPTION OF THE DRAWINGS

Various embodiments of the present invention will be discussed with reference to the appended drawings. These drawings depict only illustrative embodiments of the invention and are not to be considered limiting of its scope.

FIG. 1 is a side elevation view of an illustrative navigated surgical sizing guide according to the present invention mounted on a bone; and

FIG. 2 is a top plan view of the sizing guide of FIG. 1.

### DESCRIPTION OF THE ILLUSTRATED EMBODIMENTS

Embodiments of a navigated surgical sizing guide may be configured to make a variety of surgical measurements. For example, a navigated surgical sizing guide may be used to measure a dimension of a body part at a surgical site such as at a hip joint, knee joint, vertebral joint, shoulder joint, elbow joint, ankle joint, digital joint of the hand or foot, fracture site, tumor site, and/or other suitable surgical site. For example, the navigated surgical sizing guide may be used to measure the anterior-posterior dimension of a bone adjacent to a joint such as the femur or tibia adjacent the knee joint. Likewise, the navigated surgical sizing guide may be used to measure the medial-lateral dimension of a bone adjacent to a joint such as the femur or tibia at a knee joint. Similarly, the navigated surgical sizing guide may be used to measure the diameter of the femoral head and/or the diameter of the acetabular socket at the hip joint. The navigated surgical sizing guide may include a plurality of reference surfaces mounted for motion relative to one another to allow the reference surfaces to be positioned adjacent surgical landmarks to be measured. The reference surfaces may be mounted for three dimensional relative motion in three space, two dimensional relative motion in a plane, and/or single dimensional relative motion along a prescribed path.

The navigated surgical sizing guide may also include means for establishing a datum adjacent to a surgical site that may be referenced by subsequent surgical components. The datum may include a projection extending from the bone, a depression formed in the bone, and/or some other datum. For example, a pin, screw rail, fin, plate, dovetail, or other projection may be attached to or formed on the bone to indicate a desire anterior-posterior position. Similarly, for example, a hole, slot, dovetail, or other depression may be formed in the bone to indicate a desired anterior-posterior position.

The navigated surgical sizing guide may include tracking elements that are detectable electromagnetically, acoustically, by imaging, and/or by other suitable detection means. Furthermore, the tracking element may be active or passive. Examples of active tracking elements may include electromagnetic elements in an electromagnetic system, light emitting diodes in an imaging system, and ultrasonic emitters in an acoustic system, among others. Examples of passive tracking elements may include elements with reflective surfaces. For example, reflective spheres or discs may be attached to the orthopaedic guide and detected by an imaging system.

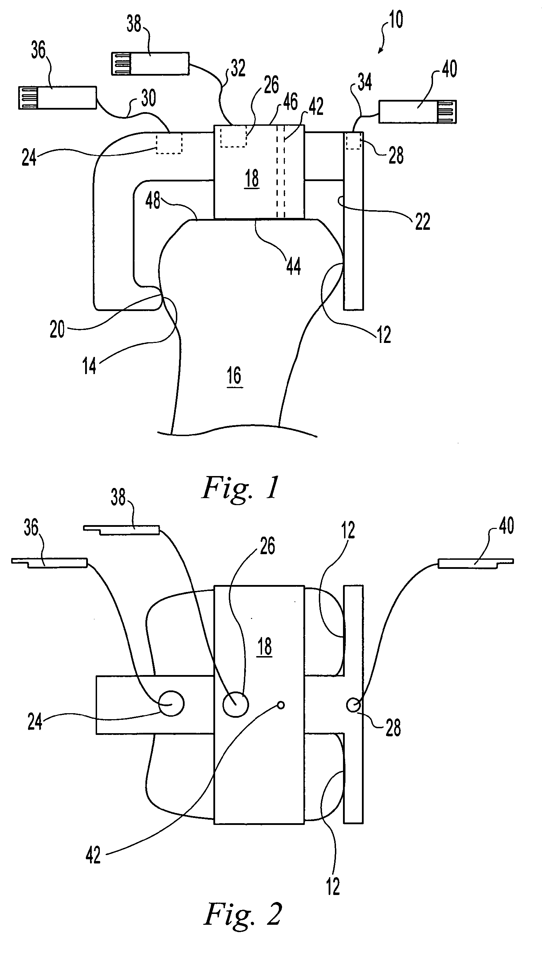

FIGS. 1 and 2 depict an illustrative navigated surgical sizing guide 10 configured to measure the anterior-posterior distance between the posterior condyles 12 and the anterior femoral cortex 14 of a femur 16 adjacent to a knee joint. The navigated surgical sizing guide 10 includes first and second reference surfaces and an optional body 18. In the illustrative navigated surgical sizing guide 10, the first reference surface includes an anterior cortex reference probe 20 and the second reference surface includes a pair of posterior condyle referencing paddles 22. The probe 20 and pair of paddles 22 are mounted for motion relative to one another to permit them to be positioned adjacent to the posterior condyles 12 and anterior femoral cortex 14. In the illustrative navigated surgical sizing guide 10, the probe 20 and pair of paddles 22 are mounted for anterior-posterior translation relative to one another. For example, the probe 20 and paddles 22 may be mounted in linear telescoping relationship to one another. Alternatively, either one or both of the probe 20 and pair of paddles 22 may be telescopically mounted to the body 18 to permit anterior-posterior adjustment of the reference surfaces relative to the body and/or one another.

One or more tracking elements 24, 26, 28, in the form of electromagnetic coils, may be mounted on the navigated surgical sizing guide 10 to provide position information to the surgical navigation system. Each of the exemplary tracking element 24, 26, 28 includes a lead 30, 32, 34 terminating in a plug 36, 38, 40 connectable to the surgical navigation system for transmitting electrical signals between the surgical navigation system and the tracking element 24, 26, 28. When the tracking element 24, 26, 28 is placed within an electromagnetic field, it generates an electrical charge that is transmitted to the surgical navigation system such that the three dimensional position and orientation of the tracking element 24, 26, 28, and thus the component to which it is attached, can be related to the surgical navigation coordinate system. For example, the surgical navigation system may include multiple sensors at known locations that receive signals from the tracking element 24, 26, 28 and feed the information to a computer. The computer may then triangulate the three dimensional position of the tracking element within the surgical navigation coordinate system. The surgical navigation system may then determine the position and orientation of the navigated surgical sizing guide 10 by detecting the position and orientation of the tracking element 24, 26, 28 and resolving the position and orientation of the navigated surgical sizing guide 10 from the known relationship between the tracking element 24, 26, 28 and the navigated surgical sizing guide 10.

If an accurate and complete computer model of the femur, or other anatomical feature to be measured, is available to the surgical navigation system, it is possible for measurements to be made in the computer model. However, if the model is incomplete or if it is desired to verify the model data, the navigated surgical sizing guide may be used to directly measure the femur.

In one configuration of the navigated surgical sizing guide 10, at least a portion of the femoral geometry is known to the surgical navigation system. The probe 20 is telescopically mounted to the body 18 and the pair of paddles 22 is fixedly attached to the body 18. A single tracking element 24 is mounted to the probe 20. In use, the femur 16 is indexed to the surgical navigation system coordinate system such as by touching an indexing probe to the bone at identified locations and relating these locations to the computerized model of the bone produced by CT scanning, MRI scanning, three dimensional X-ray, and/or other means. A geometric model of the navigated surgical sizing guide 10, for example in the form of an electronic computer aided design model, is also provided to the surgical navigation system such as by storing the model in the systems memory. The navigated surgical sizing guide 10 is then placed on the femur and positioned with the pair of paddles 22 abutting the posterior condyles 12 and the probe 20 abutting the anterior femoral cortex 14. The system can determine the position of the paddles 22 by comparing the model of the bone to the model of the navigated surgical sizing guide 10 in its memory. The surgical navigation system can determine the position of the probe 14 by detecting the tracking element 24 and resolving the probe 20 position. Now knowing the positions of the paddles 22 and probe 20, the surgical navigation system can resolve the anterior-posterior distance between the posterior femoral condyles 12 and anterior femoral cortex 14 and output the distance for surgeon reference. The system can also compare the distance to a table of known available implants and output the corresponding implant size for surgeon reference. Alternatively, a tracking element 28 may be mounted on the posterior paddles 22 and the anterior probe 20 may be the known fixed reference in the surgical navigation system.

In another configuration of the navigated surgical sizing guide 10, a pair of tracking elements may be provided to allow the system to directly measure the positions of the two tracking elements and resolve the spacing of the probe 14 and paddles 12 based on the tracking element positions. For example a first tracking element 24 may be mounted on the probe 14 and a second tracking element 28 may be mounted on the paddles 22. The probe 14 and paddles 22 are mounted for relative anterior-posterior translation. The navigated surgical sizing guide 10 is placed on the femur 16 with the probe 20 abutting the anterior femoral cortex 14 and the paddles 22 abutting the posterior femoral condyles 12. The surgical navigation system may then detect the tracking element locations and resolve the relative positions of the probe 20 and paddles 22 to determine the anterior-posterior spacing. With two tracking elements it is not necessary for the bone geometry to be known to the surgical navigation system for the system to be able to determine the anterior-posterior dimension of the bone. Alternatively, either one or both of the probe 20 and paddles 22 may be telescopically mounted on the body 18.

In another configuration of the navigated surgical sizing guide 10, the body 18 includes means for establishing a datum on the femur to indicate a desired anterior-posterior position. For example, the navigated surgical sizing guide 10 may be used to establish a datum that is later referenced by a femoral cut guide. In the illustrative navigated surgical sizing guide 10, a hole 42 extends from an anterior surface 44 to a posterior surface 46 of the body 18 to guide a drill bit to form a hole in the distal portion of the femur 16 or to guide placing a reference pin in the distal portion of the femur 16. The body 18 may be fixed relative to one of the probe 20 or paddles 22 so that the hole 42 is positioned at a constant anterior-posterior distance from the anterior femoral cortex 14 or posterior femoral condyles 12 respectively. Alternatively, the body 18 may be translatable relative to both the probe 20 and the paddles 22 and each of the probe 20, body 18, and paddles 22, may include its own tracking element 24, 26, 28. In this configuration, the probe 20 and paddles 22 are positioned and the anterior-posterior dimension of the femur is output for surgeon reference. With the navigated surgical sizing guide 10 still in position, the body 18 is translated anteriorly and posteriorly to a desired position on the bone. The desired position may be determined by preoperatively loading a bone model into the system and indicating the desired position manually, by programming the system to compare available implants to the bone model and/or measured anterior-posterior size and optimizing the position, or by some other appropriate means. The system indicates to the surgeon when the hole 42 is at the appropriate position. The surgeon may then drill a hole or position a pin as desired to mark the location. The navigated surgical sizing guide 10 may then be removed and a subsequent surgical component, such as a femoral cutting guide, may be positioned by referencing the pin or drilled hole.

The illustrative navigated surgical sizing guide 10 is shown positioned on a cut distal surface 48 of a femur 16. The navigated surgical sizing guide 10 may be used on a cut or an uncut femur. Where the navigated surgical sizing guide 10 is used to establish a datum for a subsequent femoral finishing guide, it may be desirable to have already made the distal femoral cut before positioning the datum.

Although examples of a navigated surgical sizing guide and its use have been described and illustrated in detail, it is to be understood that the same is intended by way of illustration and example only and is not to be taken by way of limitation. The invention has been illustrated in use to measure the anterior-posterior dimension of the femur and to establish a datum on the distal femur in knee replacement surgery. However, the navigated surgical sizing guide may be configured for use at other locations within a patient's body to make other measurements, to position other types of datums, and/or for use with other types of surgical components. Accordingly, variations in and modifications to the navigated surgical sizing guide and its use will be apparent to those of ordinary skill in the art, and the following claims are intended to cover all such modifications and equivalents.

## Claims

1. A navigated surgical sizing guide for use with a surgical navigation system during an orthopaedic surgical procedure to make a measurement, the navigated surgical sizing guide comprising:
first means for engaging a first position at a surgical site;
second means for engaging a second position at a surgical site, the first and second means being mounted for relative translation;
first means for being tracked by the surgical navigation system, the first means for being tracked being mounted to the first means for engaging such that the surgical navigation system is able to track the position of the first means for engaging and resolve and output the relative spacing between the first means for engaging and the second means for engaging.

2. The navigated surgical sizing guide of claim 1 further comprising:
second means for being tracked by the surgical navigation system, the second means for being tracked being mounted to the second means for engaging such that the surgical navigation system is able to track the position of the second means for engaging and resolve the relative spacing of the first means for engaging and the second means for engaging by comparing the positions of the first and second means for being tracked.

3. The navigated surgical sizing guide of claim 2 further comprising:
means for establishing a datum at a desired position relative to the surgical site such that the datum is able to be engaged by a subsequent surgical component to guide placement of the subsequent surgical component.

4. The navigated surgical sizing guide of claim 3 wherein the means for establishing a datum is mounted for translation relative to at least one of the first and second means for engaging, the navigated surgical sizing guide further comprising third means for being tracked by the surgical navigation system fixedly mounted to the means for establishing a datum, the third means for being tracked being trackable by the surgical navigation system to guide positioning of the means for establishing a datum.

5. The navigated surgical sizing guide of claim 3 wherein the means for establishing a datum comprises means for forming a projection extending from the bone.

6. The navigated surgical sizing guide of claim 3 wherein the means for establishing a datum comprises means for forming a depression in the bone.

7. The navigated surgical sizing guide of claim 1 wherein the first and second means for engaging are able to engage an anterior femoral cortex and posterior femoral condyles such that the surgical navigation system is able to resolve and output the anterior-posterior dimension of a femoral bone.

8. A navigated surgical sizing guide for use with a surgical navigation system during an orthopaedic surgical procedure to measure the size of a bone between first and second locations on the bone, the navigated surgical sizing guide comprising:
a first probe for contacting the first location on the bone, the first probe including a first tracking element trackable by the surgical navigation system; and
a second probe for contacting the second location on the bone, the second probe including a second tracking element trackable by the surgical navigation system, the first and second probes being mounted for translation relative to one another such that they may be moved between a first position in which they are relatively near one another and a second position in which they are relatively far apart, the surgical navigation system being able to track the tracking elements and resolve the distance between the first and second probes.

9. The navigated surgical sizing guide of claim 8 further comprising:
a body, the first and second probes being mounted on the body for translation relative to the body along an axis, the body including a third tracking element trackable by the surgical navigation system to determine the position of the body relative to the first and second probes, the body including means for establishing a datum on the bone for guiding subsequent surgical components.

10. The navigated surgical sizing guide of claim 9 wherein the means for establishing a datum comprises a hole extending through the body transverse to the translation axis of the first and second probes.

11. The system of claim 9 wherein the tracking elements comprise electromagnetic coils, the electromagnetic coils producing a signal detectable by the means for tracking.

12. A navigated surgical sizing guide for use with a surgical navigation system during an orthopaedic surgical procedure to measure the anterior-posterior distance between the anterior femoral cortex and the posterior femoral condyles of a femur, the navigated surgical sizing guide comprising:
a probe for contacting the anterior femoral cortex, the probe including a first tracking element trackable by the surgical navigation system; and
a paddle for contacting the posterior femoral condyles, the paddle including a second tracking element trackable by the surgical navigation system, the probe and paddle being mounted for linear translation relative to one another such that the probe and paddle may be moved between a first position in which they are relatively near one another and a second position in which they are relatively far apart, the surgical navigation system being able to track the tracking elements and resolve the distance between the probe and paddle.
